Europäisches Patentamt

European Patent Office

Office européen des brevets

(11) Publication number: **0 119 431**
**B1**

(12) **EUROPEAN PATENT SPECIFICATION**

(45) Date of publication of patent specification: **16.09.87**

(51) Int. Cl.⁴: **C 07 H 17/08**

(21) Application number: **84101239.6**

(22) Date of filing: **07.02.84**

(54) **Method of preparing erythromycin A cyclic 11,12-carbonate.**

(30) Priority: **10.02.83 PL 240514**

(43) Date of publication of application:
**26.09.84 Bulletin 84/39**

(45) Publication of the grant of the patent:
**16.09.87 Bulletin 87/38**

(84) Designated Contracting States:
**DE NL**

(56) References cited:
**US-A-3 417 077**

(73) Proprietor: **Tarchominskie Zaklady
Farmaceutyczne "Polfa"
Fleminga Strasse No. 2
PL-03-176 Warszawa (PL)**

(72) Inventor: **Biedrzycki, Marek, Dr. Ing.
Aneskazego Str. No. 2 m. 41
Warszawa (PL)**
Inventor: **Dahlig, Halina, Prof. Dr. Ing.
Nowowiejska Str. No. 22 m. 12
Warszawa (PL)**
Inventor: **Chmielnicki, Piotr
Gwardzistów Str. No. 8 m. 39
Warszawa (PL)**
Inventor: **Glabski, Tadeusz, Dr. Ing.
Narbuta Str. No. 39 m. 3
Warszawa (PL)**

(74) Representative: **Sandmair, Kurt, Dr. et al
Patentanwälte Schwabe, Sandmair, Marx
Stuntzstrasse 16
D-8000 München 80 (DE)**

The file contains technical information
submitted after the application was filed and
not included in this specification

## Description

The subject matter of the invention is a process of preparing erythromycin A cyclic 11,21-carbonate, used as an antibacterial with more advantageous properties than the parent antibiotic, simultaneously not being its prodrug. The preparation of erythromycin A cyclic 11,12-carbonate consists in a transesterification of ethylene carbonate using erythromycin A or its salts. A process for preparing erythromycin cyclic carbonate in the reaction of erythromycin with a considerable excess of ethylene carbonate in an inert solvent in the presence of alkaline catalysts, particularly of $K_2CO_3$ is known from the US—A—3 417 077. However in the described process the total reaction of the substrate is not only not complete, but considerable quantities of 10,11-anhydroerythromycin A are formed. The degree of the reacted substrate in the desired direction may be increased. According to the Polish patent specification No. 70 623 it can be done due to the use of anhydrous substrates and reduction of temperature with a simultaneous diminution of ethylene carbonate excess. According to the Polish patent specification No. 93 849 erythromycin thiocyanate or lactate may be used as starting materials.

In both cases the reaction is carried out in an aprotic solvent, advantageously in toluene. The high degree of the reacted substrate is obtained, according to the Polish patent specification No. 86 961, by the use of ethylene carbonate as solvent, constituting simultaneously the substrate in the process of transesterification.

The discussed process, independently of the way it is run, is taking place in a two-phase system, namely the liquid and the solid phase. In the mentioned Polish patent specifications the solid phase is constituted by $K_2CO_3$ as catalyst. Although the quantities of the $K_2CO_3$ catalyst are less than in the case of the U.S.A. patent specification, it is used on the average as 1 part by weight per 2 parts of erythromycin.

The disadvantage of the discussed methods consists of long duration of the reaction, favoring the formation of by-products such as e.g. of 10,11-anhydroerythromycin A, particularly at increased temperature. One of the causes of this phenomenon consists in the use of big quantities of highly alkaline inorganic catalysts in an anhydrous medium of organic solvents, in which they dissolve badly.

It has been found that the use in the reaction as catalysts of substances of the PTC (Phase Transfer Catalyst) type, such as quaternary ammonium compounds, phosphonium compounds, crown ethers or cryptands, enables to reduce considerably the quantity of the used alkaline catalyst, to shorten the duration of the transesterification reaction of the cyclic 1,2- or 1,3-diole carbonate by means or erythromycin or of its salts. In the case of introduction of definite quaternary ammonium or phosphonium compounds it even permits to eliminate from the process the alkaline catalyst. The transesterification process proceeds in dehydrated organic solvents.

Thus the invention relates to a process for the manufacture of erythromycin A cyclic 11,12-carbonate by transesterifications of cyclic 1,2-diole and 1,3-diole carbonates, using dehydrated erythromycin or its salts, in an anhydrous organic solvent, preferably selected from toluene, acetone and an anhydrous cyclic diole carbonate, characterized in that it is carried out in the presence of a phase transfer catalyst such as a quaternary ammonium compound, quaternary phosphonium compound, a crown ether or a cryptand with at least one alkali metal or earth-alkali metal salt such as bicarbonate, carbonate, hydroxide or oxide or mixtures thereof, the obtained erythromycin A cyclic 11,12-carbonate being subsequently separated and purified.

An additional advantage consists in the minimization of the appearance in the reaction products of 10,11-anhydroerythromycin A, which constitutes a decomposition product of erythromycin A cyclic 11,12-carbonate. Apart from that a diminution of the quantity of cyclic 1,2-diole carbonate (and even the application of cyclic 1,3-diole carbonate), constituting the basic reactant in the transesterification process, is advantageous in comparison with the known methods.

By the process according to the invention the transesterification reaction either of dehydrated cyclic 1,2-diole carbonates, advantageously of ethylene or propylene carbonate, or of dehydrated cyclic 1,3-diole carbonates, advantageously of trimethylene carbonate using dehydrated erythromycin or its salts, is conducted either in an anhydrous organic solvent or in an anhydrous diole carbonate, constituting simultaneously one of the reaction substrates, in the presence of the PTC type catalyst, such as a quaternary ammonium or phosphonium compound with addition of an inorganic compound.

As organic solvents are advantageously used toluene or acetone.

As quaternary ammonium compounds are used: triethylbenzylammonium chloride (TEBA), tetrabutylammonium bromide, trioctylmethylammonium chloride and as phosphonium compound tetrabutylphosphonium chloride.

It has been found advantageous to use triethylbenzylammonium chloride in an organic solvent, such as benzene or its homologues, or trioctylmethylammonium chloride in such an organic solvent as e.g. acetone or acetonitrile.

Inorganic compounds used according to the invention consist either of salts of alkali metals or of alkaline earth metals, such as sodium, potassium, lithium, calcium, barium carbonates or acid carbonates, advantageously potassium carbonate, or of hydroxides or oxides of the above mentioned metals or mixtures of these compounds.

As PTC catalysts may be also applied according to the invention crown ethers or cryptands.

The use of crown ethers in the erythromycin A cyclic 11,12-carbonate production improves the solubility of inorganic cations, increasing thus the effectiveness of the catalysis by basic inorganic salts. The use of crown ethers containing 12 carbon atoms and 6 oxygen atoms in the basic moiety, especially of 1,4,7,13,16-hexaoxacyclooctadecane is particularly advantageous. Equal results are obtained with cryptands, advantageously 4,7,13,16,21,24 - hexaoxa - 1,10 - diazabicyclo - [8,8,8] - hexacosane.

Should as PTC catalyst a quaternary ammonium or phosphonium compound be used, an equilibrium in the liquid phase would take place between the initial ammonium or phosphonium salt and the quaternary compound newly formed in the reaction medium containing the anion derived from the alkali metal or from the alkaline earth metal compound. According to the invention it is particularly important to run the transesterification reaction in the presence of, as starting materials either quaternary ammonium hydroxides alone or their salts with weak acids, advantageously carbonates, or analogous phosphonium compounds, although this process is in a certain degree limited. In such a transesterification process both the quaternary ammonium compound and the phosphonium compound constitute independent catalysts.

The use of PTC catalysts brings about a considerable increase of concentration of the catalyst in the organic solvent, which may also provoke the formation of undesirable degradation products. It is therefore necessary to choose properly the concentration, the kinds of catalysts and the organic solvent, as well as the parameters of the process in order to secure its most effective course from the point of view of industrial production.

Pursuing the process according to the present invention, erythromycin or the intermediate product obtained during its isolation, as for example erythromycin thiocyanate or lactate may be used in the discussed transesterification process.

Erythromycin A cyclic 11,12-carbonate obtained according to the invention has a 6,9-hemiacetal structure (Slawinski W., Bojarska-Dahlig H., Gąbski T., Dzięgielewska I., Biedrzycki M., Naperty S., Rec. trav. Chim. 94, 236, 1975). Crystallized from the 50% aqueous solution of acetone its melting point is 219°—224°C, its specific rotatory power $[\alpha]_D^{21}=-54°$ (c=2, dioxan). Its total formula: $C_{38}H_{65}NO_{14}$. (M=759,95).

Analysis for the formula:
calculated: 60.33% C; 8.78% H; 1.81% N;
obtained: 60.0% C; 8.4% H; 1.8% N.

Molecular mass: 760
IR: 1815 (carbonate), 1755 (lactone), 1740 lactone-hydrogen bond) $cm^{-1}$.
$^1H$ NMR=3.29 (OMe), 2.26 (MNe$_2$), 1.59 (Me), 1.35 (Me).
Mass spectrum: molecular ion 761.

Example I.

100.0 g (0.136 mole) of erythromycin containing less than 2% water were dissolved in 400 $cm^3$ of toluene and the obtained solution azeotropically dried, 8.0 g (0.058 mole) of potassium carbonate, 15.0 g (0.15 mole) of potassium bicarbonate, 2.0 g (0.0088 mole) of triethylbenzylammonium chloride and 40.0 g (0.454 mole) of ethylene carbonate were subsequently added. The whole was stirred at temperature of 40°C during 6 hours and the course of the reaction controlled with thin-layer chromatography (TLC). The reaction mixture was filtered and ethanol added to the filtrate. The obtained solution was subjected to azeotropic distillation under reduced pressure and the remaining product crystallized from the ethanol—water mixture, 85.9 g (83% of the theoretical yield) of erythromycin A cyclic 11,12-carbonate with its melting point of 219—223°C were obtained.

The product was identified by HPLC (High Performance Liquid Chromatography). The HPLC (hemiacetal form) analysis was performed with the application of the method of reversed phases in a column filled with LiChrosorb RP-2, its grain size being 5 μm, with the use as mobile phase of the system acetonitrile—aqueous solution of ammonium acetate of the concentration of 0,2 mole/litre—water (6:1:3) and UV detection at λ=218 nm. Retention time $T_R$ is 10.2 minutes.

Example II.

70.0 g (0.0784 mole) of dry erythromycin thiocyanate were suspended in a mixture of 200 $cm^3$ of toluene and 100 $cm^3$ of water. The whole was warmed up to temperature of 50°C and a 10% aqueous solution of NaOH added to obtain pH=9.5. The phases were subsequently separated and the organic layer dried. To the organic solution, after filtering off the desiccant, 5.28 g (0.036 mole) of potassium carbonate, 1.0 g (0.0031 mole) of tetrabutylammonium bromide and 22.9 g (0.22 mole) of propylene carbonate were added, whereupon the mixture was stirred at temperature of 20°C during 10 hours. The reaction mixture was then washed with water. Toluene was distilled off from the organic phase under reduced pressure and the dry residue crystallized from the acetone—water mixture, 44.0 g (85% of the theoretical yield) of the product as in example I were thus obtained. The erythromycin A cyclic 11,12-carbonate was identified by the HPLC method. Retention time $T_R$, as well as its melting point as in example I.

Example III.

50.0 g (0.068 mole) of dry erythromycin were dissolved in 200 $cm^3$ of toluene and to the obtained solution 5.0 g (0.024 mole) of triethylbenzylammonium hydroxide and 20.0 g (0.228 mole) of ethylene carbonate added. The whole was stirred at a temperature of 50°C during 10 hours. The reaction mixture was filtered and the antibiotic separated from the filtrate in the way as in example II, obtaining thus 37.7 g (74% of the theoretical yield) of the product with its melting point of 200—224°C. Retention time $T_R$ as in example I.

Example IV.

To the dried solution of 25.0 g (0.034 mole) of erythromycin in 150 cm$^3$ of acetone 7.6 g (0.05 mole) of barium oxide and 2.5 g (0.0062 mole) of trioctylmethylammonium chloride were added and stirred at temperature of 40°C during 10 hours. The precipitate was removed, water subsequently dropped in into the organic filtrate at its continuous stirring, 18.4 g (71% of the theoretical yield) of the product as in example I were thus obtained.

Example V.

25.0 g (0.034 mole) of dry erythromycin were dissolved in 100 cm$^3$ of toluene and to the obtained solution 2.65 g (0.018 mole) of potassium carbonate, 0.5 g (0.0022 mole) of triethylbenzylammonium chloride and 13.7 g (0.136 mole) of trimethylene carbonate subsequently added. The whole was stirred at temperature of 40°C during 12 hours. The reaction mixture was filtered and the antibiotic separated from the obtained toluene phase in the same way as in example I. Thus 18.2 g (70% of the theoretical yield) of the product with its melting point of 219°—22°C and the retention time $T_R$ as in example I were obtained.

Example VI.

10.0 g (0.0136 mole) of azeotropically dried erythromycin dissolved in 35.2 g (0.40 mole) of liquid ethylene carbonate, stirred at temperature of 50°C, 0.3 g (0.0011 mole) of triethylbenzylammonium chloride and 1.0 g (0.0072 mole) of potassium carbonate were added to the obtained solution and stirred at the same temperature during 3 hours. The reaction mixture was diluted with water and thus 8.2 g (79% of the theoretical yield) of the product with its melting point of 219°—224°C and the retention time $T_R$ as in example I obtained.

Example VII.

25.0 g (0.034 mole) of dry erythromycin were dissolved in 100 cm$^3$ of toluene and to the solution 3.7 g (0.027 mole) of potassium carbonate, 0.8 g (0.0027 mole) of tetrabutylphosphonium chloride and 12.5 g (0.142 mole) of ethylene carbonate subsequently added. The whole was stirred at temperature of 30°C during 10 hours. The reaction mixture was filtered, the antibiotic separated from the filtrate in the same way as in example I and thus 18.5 g (71% of the theoretical yield) of the product with its melting point of 219°—221°C and the retention time $T_R$ as in example I obtained.

Example VIII.

25.0 g (0.034 mole) of azeotropically dried erythromycin were dissolved in 100 cm$^3$ of toluene, to the solution 2.5 g (0.018 mole) of potassium carbonate, 0.075 g (0.0003 mole) of 1,4,7,10,13,16-hexaoxacyclooctadecane and 12.5 g (0.142 mole) of ethylene carbonate subsequently added and stirred at temperature of 20°C during 12 hours. When reaction was finished, the reaction mixture and subsequently the remaining toluene layer were washed with water. The antibiotic was separated from the organic phase as in example II and thus 19.5 (75% of the theoretical yield) of the product with its melting point of 220°—223°C and the retention time $T_R$ as in example I obtained.

Example IX.

25.0 g (0.034 mole) of dry erythromycin were dissolved in 100 cm$^3$ of toluene, subsequently into the obtained solution 2.5 g (0.018 mole) of potassium carbonate, 0.15 g (0.0004 mole) of 4,7,13,16,21,24-hexaoxa-1,10-diazabicyclo[8,8,8]hexacosane and 12.0 g (0.136 mole) of ethylene carbonate added and stirred at temperature of 50°C during 10 hours. The reaction mixture is filtered, the antibiotic separated from the filtrate as in example I and 19.7 g (76% of the theoretical yield) of the product as in example I thus obtained.

**Claims**

1. A process for the manufacture of erythromycin A cyclic 11,12-carbonate by transesterifications of cyclic 1,2-diole and 1,3-diole carbonates, using dehydrated erythromycin or its salts, in an anhydrous organic solvent, preferably selected from toluene, acetone and an anhydrous cyclic diole carbonate, characterized in that it is carried out in the presence of a phase transfer catalyst such as a quaternary ammonium compound, quaternary phosphonium compound, a crown ether or a cryptand with at least one alkali metal or earth-alkali metal salt such as bicarbonate, carbonate, hydroxide or oxide or mixtures thereof, the obtained erythromycin A cyclic 11,12-carbonate being subsequently separated and purified.

2. A process according to claim 1, characterized in that in the process of transesterification there are used cyclic 1,2-diole carbonates, advantageously ethylene carbonate or propylene carbonate.

3. A process according to claim 1, characterized in that in the process of transesterification there are used cyclic 1,3-diole carbonates, advantageously trimethylene carbonate.

4. A process according to claim 1 characterized in that as a quaternary ammonium compound triethylbenzylammonium chloride, tetrabutylammonium bromide, trioctylmethylammonium chloride with at least one alkali metal or earth-alkali metal bicarbonate, carbonate, hydroxide or oxide or mixtures thereof is used.

5. A process according to claim 1 characterized in that as a quaternary phosphonium compound tetrabutylphosphonium chloride with at least one alkali metal or earth-alkali metal bicarbonate, carbonate, hydroxide or oxide or mixtures thereof is used.

6. A process according to claim 1, characterized in that as a crown ether there is advantageously used ether containing 12 carbon atoms and 6 oxygen atoms, named 1,4,7,10,13,16-hexaoxacyclooctadecane.

7. A process according to claim 1, characterized in that as a cryptand there is advantageously used 4,7,13,16,21,24 - hexaoxa - 1,10 - diazabicyclo - [8,8,8]hexacosane.

8. A process for the manufacture of erythromycin A cyclic 11,12-carbonate by transesterifications of cyclic 1,2-diole and 1,3-diole carbonates using dehydrated erythromycin or its salts, in an anhydrous organic solvent, preferably selected from toluene, acetone and an anhydrous cyclic diole carbonate, characterized in that it is carried out in the presence of an independent catalyst such as quaternary ammonium hydroxide or carbonate or quaternary phosphonium hydroxide or carbonate, the obtained erythromycin A cyclic 11,12-carbonate being subsequently separated and purified.

9. A process according to claim 8 characterized in that as cyclic 1,2-diole carbonates ethylene carbonate or propylene carbonate is used.

10. A process according to claim 8 characterized in that as cyclic 1,3-diole carbonate trimethylene carbonate is used.

## Patentansprüche

1. Verfahren zur Herstellung von Erythromycin A-11,12 cyclischem Carbonat durch Umesterung von cyclischem 1,2-Diol- und 1,3-Diolcarbonaten bei dem man dehydriertes Erythromycin oder seine Salze in einem wasserfreien organischen Lösungsmittel verwendet, wobei das Lösungsmittel vorzugsweise aus Toluol, Aceton und wasserfreiem cyclischem Diolcarbonat ausgewählt ist, dadurch gekennzeichnet, daß man das Verfahren in Gegenwart eines Phasentransfer-Katalysators wie einer quaternären Ammoniumverbindung, quaternären Phosphoniumverbindung, einem Kronenether oder einem Kryptand mit zumindest einem Alkali- oder Erdalkalimetallsalz wie Bicarbonat, Carbonat, Hydroxid oder Oxid oder Mischungen davon, durchführt und das erhaltene Erythromycin A-11,12-cyclische Carbonat anschließend abtrennt und reinigt.

2. Verfahren nach Anspruch 1, bei dem man im Umesterungsverfahren cyclische 1,2-Diolcarbonate, vorzugsweise Ethylencarbonat oder Propylencarbonat, verwendet.

3. Verfahren nach Anspruch 1, bei dem man im Umesterungsverfahren cyclische 1,3-Diolcarbonate, vorzugsweise Trimethylencarbonat, verwendet.

4. Verfahren nach Anspruch 1, bei dem man als quaternäre Ammoniumverbindung Triethylbenzylammoniumchlorid, Tetrabutylammoniumbromid, Trioctylmethylammoniumchlorid mit mindestens einem Alkali- oder Erdalkalimetallbicarbonat, carbonat, -hydroxid oder -oxid oder Mischungen davon, verwendet.

5. Verfahren nach Anspruch 1, bei dem man als quaternäre Phosphoniumverbindung Tetrabutylphosphoniumchlorid mit zumindest einem Alkalimetall oder Erdalkalimetallbicarbonat, -carbonat, -hydroxid oder -oxid oder Mischungen davon, verwendet.

6. Verfahren nach Anspruch 1, bei dem man als Kronenether vorzugsweise einen Ether mit 12 Kohlenstoffatomen und 6 Sauerstoffatomen mit der Bezeichnung 1,4,7,10,13,16-Hexaoxacyclooctadecan verwendet.

7. Verfahren nach Anspruch 1, bei dem man als Kryptand vorzugsweise 4,7,13,16,21,24-Hexaoxa-1,10-diazabicyclo-[8,8,8]hexacosan verwendet.

8. Verfahren zur Herstellung von Erythromycin A-11,12-cyclischem-Carbonat durch Umesterung von cyclischem 1,2-Diol- und 1,3-Diol-carbonaten unter Verwendung von dehydriertem Erythromycin oder seinen Salzen in einem wasserfreien organischem Lösungsmittel, das vorzugsweise aus Toluol, Aceton und einem wasserfreien cyclischem Diolcarbonat ausgewählt ist, dadurch gekennzeichnet, daß man das Verfahren in Gegenwart eines Einzelkatalysators wie quaternärem Ammoniumhydroxid oder -carbonat oder quaternärem Phosphoniumhydroxid oder -Carbonat durchführt und das erhaltene Erythromycin A-11,12-cyclische-Carbonat anschließend abtrennt und reinigt.

9. Verfahren nach Anspruch 8, bei dem man als cyclisches 1,2-Diolcarbonat, Ethylencarbonat oder Propylencarbonat verwendet.

10. Verfahren nach Anspruch 8, bei dem man als cyclisches 1,3-Diolcarbonat Trimethylencarbonat verwendet.

## Revendications

1. Procédé de fabrication du 11,12-carbonate cyclique d'érythromycine A par transesterification des

5

carbonates cycliques de 1,2-diol et 1,3-diol en utilisant l'erythromycine déshydraté ou ses sels, dans un solvant organique anhydre, choisi de préférence parmi le toluène, l'acétone et un carbonate de diol cyclique anhydre, caractérisé en ce qu'il est effectué en présence d'un catalyseur de transfert de phase tel qu'un composé d'ammonium quaternaire, un compose de phosphonium quaternaire, un éther couronne ou un cryptate, avec au moins un sel de métal alcalin ou alcalino-terreux, tel qu'un bicarbonate, carbonate, hydroxyde ou oxyde ou leurs mélanges, le 11,12-carbonate cyclique d'érythromycine A obtenu étant ensuite separé et purifié.

2. Procédé selon la revendication 1, caractérisé en ce que dans le procédé de transestérification, on utilise des carbonates cycliques de 1,2-diol, de préférence le carbonate d'éthylène ou le carbonate de propylène.

3. Procédé selon la revendication 1, caractérisé en ce que dans le procédé de transestérification, on utilise des carbonates cycliques de 1,3-diol, de préférence le carbonate de triméthylène.

4. Procédé selon la revendication 1, caractérisé en ce que comme composé d'ammonium quaternaire on utilise le chlorure de triéthylbenzylammonium le bromure de tétrabutylammonium, le chlorure de trioctylméthylammonium avec au moins un bicarbonate, carbonate, hydroxyde ou oxyde de métal alcalin ou alcalino-terreux, ou leurs mélanges.

5. Procédé selon la revendication 1, caractérisé en ce que comme composé de phosphonium quaternaire on utilise le chlorure de tétrabutylphosphonium, avec au moins un bicarbonate, carbonate, hydroxyde ou oxyde de métal alcalin ou alcalino-terreux, ou leurs mélanges.

6. Procédé selon la revendication 1, caractérisé en ce que comme éther couronne on utilise de préférence un éther contenant 12 atomes de carbone et 6 atomes d'oxygène, dénommé 1,4,7,10,13,16-hexaoxacyclooctadécane.

7. Procédé selon la revendication 1, caractérisé en ce que comme cryptate on utilise de préférence le 4,7,13,16,21,24-hexaoxa-1,10-diazabicyclo-[8,8,8]hexacosane.

8. Procédé de fabrication du 11,12-carbonate cyclique d'érythromycine A par transestérification des carbonates cycliques de 1,2-diol et de 1,3-diol, en utilisant l'érythromycine déshydraté ou ses sels, dans un solvant organique anhydre, choisi de préférence parmi le toluène, l'acétone et un carbonate de diol cyclique anhydre, caractérisé en ce qu'il est effectué en présence d'un catalyseur indépendant tel qu'un hydroxyde ou carbonate d'ammonium quaternaire ou un hydroxyde ou carbonate de phosphonium quaternaire, le 11,12-carbonate cyclique d'érythromycine A obtenu étant ensuite séparé et purifié.

9. Procédé selon la revendication 8, caractérisé en ce que l'on utilise comme carbonate cyclique de 1,2-diol, le carbonate d'éthylène ou le carbonate de propylène.

10. Procédé selon la revendication 8, caractérisé en ce que l'on utilise comme carbonate cyclique de 1,3-diol, le carbonate de triméthylène.